(19) Europäisches Patentamt
European Patent Office
Office européen des brevets

(11) EP 4 768 467 A1

(12) EUROPEAN PATENT APPLICATION
published in accordance with Art. 153(4) EPC

(43) Date of publication:
**01.07.2026 Bulletin 2026/27**

(21) Application number: **24856790.1**

(22) Date of filing: **20.08.2024**

(51) International Patent Classification (IPC):
***C07C 69/84*** *(2006.01)* ***C07C 67/52*** *(2006.01)*
***C07C 67/54*** *(2006.01)* ***C07C 67/03*** *(2006.01)*
***C08J 11/24*** *(2006.01)*

(52) Cooperative Patent Classification (CPC):
**C07C 67/03; C07C 67/52; C07C 67/54; C07C 69/84; C08J 11/24;** Y02W 30/62

(86) International application number:
**PCT/KR2024/012341**

(87) International publication number:
**WO 2025/042179 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.08.2023 KR 20230110791**

(71) Applicant: **SK Chemicals Co., Ltd.**
**Seongnam-si, Gyeonggi-do 13494 (KR)**

(72) Inventors:
- **KIM, Ji-Hun**
  **Seongnam-si, Gyeonggi-do 13494 (KR)**
- **PARK, Kwang-Woo**
  **Seongnam-si, Gyeonggi-do 13494 (KR)**
- **RYU, Gayeong**
  **Seongnam-si, Gyeonggi-do 13494 (KR)**
- **LEE, Joong Ki**
  **Seongnam-si, Gyeonggi-do 13494 (KR)**

(74) Representative: **Haseltine Lake Kempner LLP**
**One Portwall Square**
**Portwall Lane**
**Bristol BS1 6BH (GB)**

# (54) RECYCLED RAW MATERIAL COMPOSITION AND METHOD FOR MANUFACTURING SAME

(57) The present invention relates to a recycled raw material composition and a method for manufacturing the recycled raw material composition, the recycled raw material composition comprising recycled bis(2-hydroxyethyl) terephthalate formed by depolymerization of waste polyester, wherein the peak area ratio of an acetate-based ester compound, as determined by high-performance liquid chromatography (HPLC) analysis, is 1.0% or less. The recycled raw material composition can exhibit high purity and high quality due to a reduced content of impurities.



Processed by Luminess, 75001 PARIS (FR)

## Description

### Technical Field

The present invention relates to a recycled raw material composition obtained through a recycling process of waste polyester, which can have high purity and high quality with minimized impurities, and a process for preparing the recycled raw material composition.

### Background Art

Polyester, among polymers commonly used in modern life, is widely used as a material for containers for beverages and food, various packaging films, interior and exterior materials such as panels, shelves, and partitions, and the like by virtue of its excellent mechanical strength, thermal resistance, transparency, and gas barrier properties.

As a result, waste of plastics such as polyester is generated at an unmanageable level every year. Recently, countries around the world have prepared regulations and plans for recycling waste plastic resources, including waste polyester.

Although physical or chemical recycling methods are used as methods of recycling waste polyester, physical recycling methods cannot guarantee purity and, therefore, are not widely adopted. Meanwhile, in chemical recycling methods, the ester bond of a waste polyester is severed to depolymerize it. Reactions such as glycolysis, hydrolysis, methanolysis, and aminolysis are used. Glycolysis among the above is to decompose waste polyester by adding a glycol such as ethylene glycol or diethylene glycol. A product comprising mainly bis(2-hydroxyethyl) terephthalate (BHET) is obtained. The bis(2-hydroxyethyl) terephthalate contained in the product may be used as a raw material for preparing unsaturated polyester or ester polyol after the crystallization or purification thereof.

However, since the product comprising bis(2-hydroxyethyl) terephthalate (BHET) has a relatively high content of impurities, there is a limit to obtaining bis(2-hydroxyethyl) terephthalate (BHET) with high purity and high quality therefrom.

### Disclosure of Invention

### Technical Problem

In general, a recycled raw material composition obtained as a result of the depolymerization reaction of waste polyester comprises, in addition to bis(2-hydroxyethyl) terephthalate as a useful recycled raw material, oligomers such as dimers and trimers; diethylene glycol ester compounds derived from diethylene glycol (DEG); acetate-based ester compounds obtained by the use of an acetate catalyst; or bis(2-hydroxyethyl) terephthalate (BHET) analogues, as impurities, which is a factor that reduces the purity and quality of the recycled raw material composition.

In particular, the present inventors have confirmed that diethylene glycol ester compounds, acetate-based ester compounds, and BHET analogues, among the above impurities, lower the purity and quality of bis(2-hydroxyethyl) terephthalate contained in the recycled raw material composition; as a result, when a polymer is prepared using the recycled bis(2-hydroxyethyl) terephthalate, the polymerization degree and thermal resistance properties of the polymer are deteriorated.

To solve this problem, the present inventors have confirmed that when the polarity of the solvent is controlled during the depolymerization process of waste polyester, and a crystallization process is performed, the content of impurities such as diethylene glycol ester compounds, acetate-based ester compounds, and BHET analogues in the recycled raw material composition is adjusted to a specific range; as a result, a polymer having improved polymerization degree and thermal resistance properties is obtained, thereby completing the present invention.

Accordingly, an object of the present invention is to provide a recycled raw material composition with a minimized content of impurities, which can secure the polymerization degree and thermal resistance properties of a polymer material at a target level, and a process for preparing the same.

### Solution to Problem

In order to accomplish the above object, the present invention provides a recycled raw material composition, which comprises bis(2-hydroxyethyl) terephthalate formed by the depolymerization of waste polyester and has a peak area fraction of an acetate-based ester compound of 1.0% or less when analyzed by high-performance liquid chromatography (HPLC).

In addition, the present invention provides a process for preparing a recycled raw material composition, which comprises (1) depolymerizing waste polyester by a glycolysis reaction to obtain a reactant comprising crude bis(2-hydroxyethyl) terephthalate (crude-BHET); (2) treating the reactant with an ion-exchange resin; (3) adding water to the

reactant obtained by treatment with the ion-exchange resin to adjust the polarity of the solvent contained in the reactant; (4) cooling the reactant to which water has been added to crystallize it; and (5) performing pressurized filtration of the crystallized material obtained through the crystallization to obtain a product comprising recycled bis(2-hydroxyethyl) terephthalate.

## Advantageous Effects of Invention

In the present invention, the polarity of a solvent used in the depolymerization procedure of waste polyester is controlled, whereby the separation of impurities (e.g., acetate-based ester compounds, diethylene glycol ester compounds, BHET analogues, BHET oligomers, and the like) and purification are optimized. As a result, a recycled raw material composition with a minimized content of impurities can be obtained. Accordingly, the present invention can provide bis(2-hydroxyethyl) terephthalate with high purity and high quality as a recycled raw material (polymerization raw material) from the recycled raw material composition.

As the present invention provides bis(2-hydroxyethyl) terephthalate with high purity and high quality by depolymerizing waste polyester as described above, not only can the resource be recycled, but it can also contribute to the preparation of a polymer (e.g., a recycled polyester resin) having excellent thermal resistance properties and color characteristics using the bis(2-hydroxyethyl) terephthalate as a recycled raw material (polymerization raw material).

## Best Mode for Carrying out the Invention

Hereinafter, the present invention will be described in detail. The present invention herein is not limited to the disclosures given below, but it may be modified into various forms as long as the gist of the invention is not changed.

In the present specification, the term "comprising" is intended to specify a particular characteristic, region, step, process, element, and/or component. It does not exclude the presence or addition of any other characteristic, region, step, process, element, and/or component, unless specifically stated to the contrary.

Throughout the present specification, the terms first, second, and the like are used to describe various components. But the components should not be limited by the terms. The terms are used for the purpose of distinguishing one element from another.

All numbers and expressions related to the quantities of components, reaction conditions, and the like used herein are to be understood as being modified by the term "about" unless otherwise indicated.

In the present specification, a singular expression is interpreted to cover a plural number as well unless otherwise specified in the context.

## Recycled raw material composition

The recycled raw material composition according to the present invention comprises bis(2-hydroxyethyl) terephthalate formed by the depolymerization of waste polyester and has a peak area fraction of an acetate-based ester compound of 1.0% or less when analyzed by high-performance liquid chromatography (HPLC). The recycled raw material composition according to the present invention has a content of an acetate-based ester compound as an impurity controlled to a specific range of 1.0% or less; thus, a recycled raw material obtained therefrom, i.e., recycled bis(2-hydroxyethyl) terephthalate, has high purity. When a polymer (e.g., a recycled polyester resin) is prepared using the same, it is possible to prevent the decrease in thermal resistance properties (Tm, Tg) due to an increase in diethylene glycol units (DEG units) or the decrease in polymerization degree due to the action of terminator groups. As a result, it is possible to provide a polymer having excellent thermal resistance properties, color characteristics, and the like.

The acetate-based ester compound contained in the recycled raw material composition may be a by-product of an acetate, which is employed as a catalyst during the depolymerization of waste polyester by a glycolysis reaction. Specifically, referring to the following Reaction Scheme 1, acetic acid (AA) derived from an acetate, which is used as a catalyst, may react with ethylene glycol (EG) to produce an acetate-based compound such as 2-hydroxyethyl acetate (HA) and water ($H_2O$). In such an event, since 2-hydroxyethyl acetate (HA) has a boiling point similar to that of ethylene glycol (EG), it is not easily filtered out as an impurity during the recovery and reuse of ethylene glycol (EG). As a result, as the process is continued, by-products such as an acetate-based ester compound are accumulated. That is, referring to the following Reaction Scheme 2, 2-hydroxyethyl acetate (HA) may be subjected to a transesterification reaction with bis(2-hydroxyethyl) terephthalate (BHET) to produce an ester compound such as 2-hydroxyethyl(2-acetoxyethyl) terephthalate (HAET) and ethylene glycol (EG).

[Reaction Scheme 1]

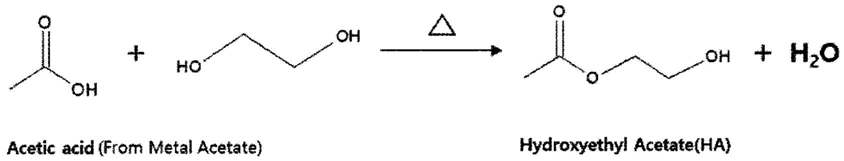


[Reaction Scheme 2]

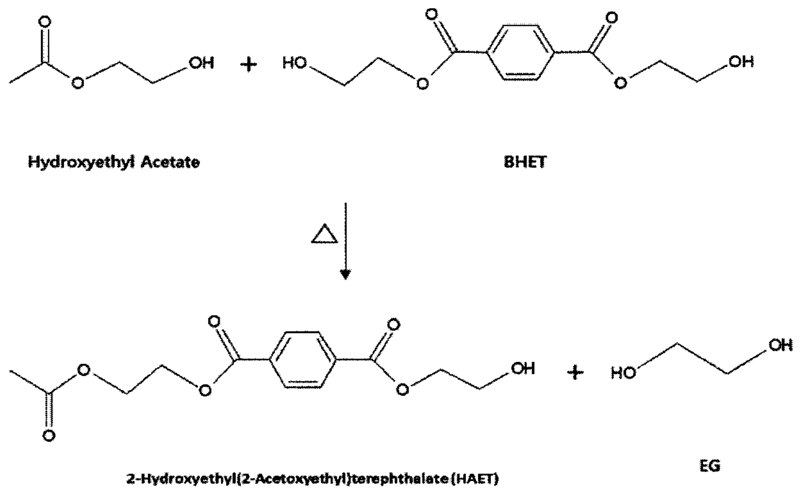


The acetate-based ester compound (e.g., 2-hydroxyethyl(2-acetoxyethyl) terephthalate (HAET)) thus produced may serve as an impurity (e.g., reduction in BHET purity, production of HA, and acting as a terminator group). The recycled raw material composition according to the present invention has a very low content of the acetate-based ester compound. As a result, recycled bis(2-hydroxyethyl) terephthalate (BHET) with high purity can be provided.

Specifically, the recycled raw material composition according to the present invention may have a peak area fraction of an acetate-based ester compound (HA-ester) of 0.9% or less, 0.8% or less, 0.7% or less, 0.6% or less, 0.5% or less, 0.4% or less, 0.3% or less, 0.2% or less, 0.1% or less, less than 0.1%, 0.09% or less, 0.08% or less, 0.07% or less, 0.05% or less, 0.04% or less, or 0.03% or less, when analyzed by high-performance liquid chromatography (HPLC). For example, the peak area fraction of an acetate-based ester compound (HA-ester) may be 0 to 1.0%, greater than 0 to 0.9%, greater than 0 to 0.5%, greater than 0 to 0.3%, greater than 0 to 0.1%, greater than 0 to 0.09%, or 0.01 to 0.09%.

Meanwhile, the recycled raw material composition according to the present invention may comprise impurities such as bis(2-hydroxyethyl) terephthalate (BHET) analogues (isomers), bis(2-hydroxyethyl) terephthalate (BHET) oligomers (dimers, trimers, and the like), and diethylene glycol ester compounds, in addition to the acetate-based ester compound. In the present invention, the content of these impurities is also controlled to a specific range.

Specifically, according to the present invention, when the recycled raw material composition is analyzed by high-performance liquid chromatography (HPLC), the peak area fraction of a compound comprising bis(2-hydroxyethyl) isophthalate, which is a BHET analogue, may be 1.0% or less. The compound comprising bis(2-hydroxyethyl) isophthalate may be a compound composed of bis(2-hydroxyethyl) isophthalate alone. The bis(2-hydroxyethyl) isophthalate may be an impurity formed when waste polyester comprising an isophthalic acid unit (IPA unit) is depolymerized by a glycolysis reaction (see the following Reaction Scheme 3).

[Reaction Scheme 3]

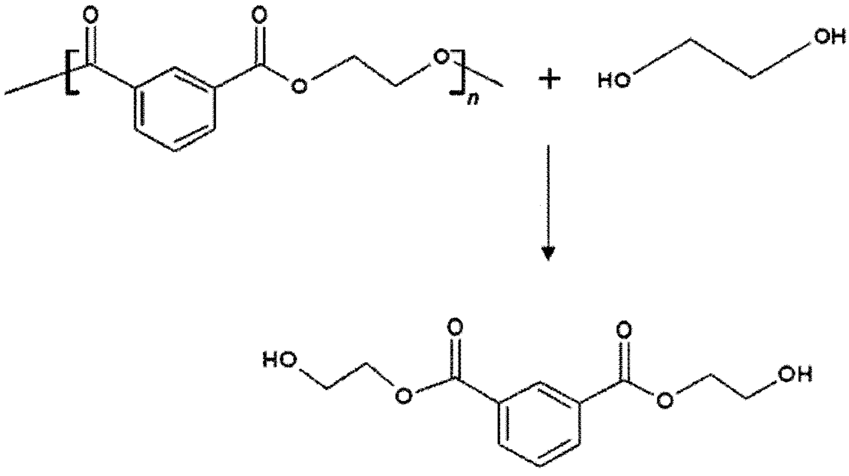

BHEI

The bis(2-hydroxyethyl) isophthalate thus produced may serve as an impurity. When a polymer is prepared using a recycled raw material composition comprising the same, the melting point (Tm) of the polymer prepared therefrom is drastically reduced, thereby significantly reducing the thermal resistance properties of the polymer. The recycled raw material composition according to the present invention has a very low content of the bis(2-hydroxyethyl) isophthalate. As a result, a polymer with excellent thermal resistance properties can be provided.

Specifically, the recycled raw material composition according to the present invention may have a peak area fraction of a compound comprising bis(2-hydroxyethyl) isophthalate (BHEI) of 0.98% or less, 0.95% or less, 0.93% or less, 0.92% or less, 0.9% or less, 0.7% or less, 0.6% or less, 0.5% or less, 0.48% or less, 0.46% or less, 0.43% or less, 0.4% or less, 0.38% or less, 0.35% or less, 0.33% or less, or 0.3% or less, when analyzed by high-performance liquid chromatography (HPLC). For example, the peak area fraction of a compound comprising bis(2-hydroxyethyl) isophthalate (BHEI) may be 0 to 1.0%, greater than 0 to 0.99%, greater than 0 to 0.97%, greater than 0 to 0.92%, greater than 0 to 0.6%, greater than 0 to 0.55%, or 0.01 to 0.35%.

According to the present invention, the recycled raw material composition may have a peak area fraction of diethylene glycol ester compounds of 2.0% or less in total (the sum of the peak area fractions of diethylene glycol ester compounds is 2.0% or less), when analyzed by high-performance liquid chromatography (HPLC). The diethylene glycol ester compounds may be produced by a transesterification reaction between diethylene glycol and bis(2-hydroxyethyl) terephthalate (BHET) during the procedure of depolymerizing waste polyester by a glycolysis reaction. The diethylene glycol ester compounds may specifically comprise 2-hydroxyethyl[2-(2-hydroxyethoxy)ethyl] terephthalate (DEG-ester-1) represented by the following Formula 1 and bis[2-(2-hydroxyethoxy)ethyl]benzene-1,4-dicarboxylate (DEG-ester-2) represented by the following Formula 2.

[Formula 1]

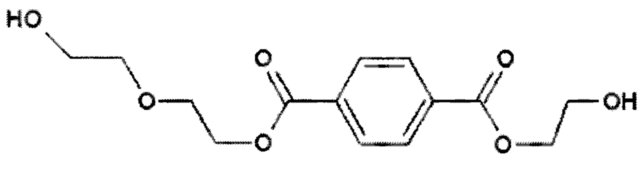

[Formula 2]

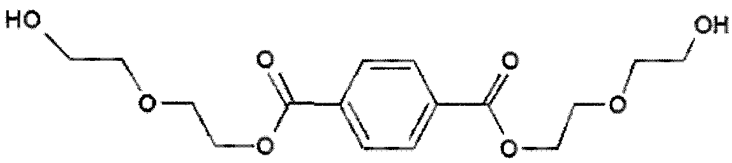

Specifically, the recycled raw material composition according to the present invention may have a peak area fraction of diethylene glycol ester compounds (DEG-esters) (e.g., the peak area fraction of DEG-ester-1 and the peak area fraction of DEG-ester-2) of 1.99% or less, 1.95% or less, 1.93% or less, 1.9% or less, 1.88% or less, 1.85% or less, 1.83% or

less, 1.8% or less, 1.78% or less, 1.77% or less, 1.75% or less, 1.73% or less, 1.7% or less, 1.65% or less, 1.63% or less, or 1.6% or less, in total, when analyzed by high-performance liquid chromatography (HPLC). For example, the peak area fraction of diethylene glycol ester compounds (DEG-esters) may be 0 to 1.99%, greater than 0 to 1.95%, greater than 0 to 1.85%, greater than 0 to 1.8%, greater than 0 to 1.7%, greater than 0 to 1.6%, or 0.01 to 1.5%, in total.

According to the present invention, the recycled raw material composition may have a peak area fraction of oligomers of dimers or higher of 2.0% or less in total (the sum of the peak area fractions of oligomers of dimers or higher is 2.0% or less), when analyzed by high-performance liquid chromatography (HPLC). The oligomers of dimers or higher may specifically be a dimer of bis(2-hydroxyethyl) terephthalate (BHET), a trimer of bis(2-hydroxyethyl) terephthalate (BHET), or a combination thereof. The oligomers may have a molecular weight of 2,000 g/mole or less (e.g., 1,000 to 2,000 g/mole).

Specifically, the recycled raw material composition according to the present invention may have a peak area fraction of oligomers of dimers or higher (e.g., the peak area fraction of dimers and the peak area fraction of trimers) of 1.5% or less, 1.3% or less, 1.0% or less, less than 1.0%, 0.95% or less, 0.9% or less, 0.85% or less, 0.8% or less, 0.75% or less, 0.7% or less, 0.65% or less, 0.6% or less, 0.55% or less, 0.5% or less, 0.45% or less, 0.4% or less, 0.39% or less, 0.35% or less, or 0.3% or less, in total, when analyzed by high-performance liquid chromatography (HPLC). For example, the peak area fraction of oligomers of dimers or higher may be 0 to 1.0%, greater than 0 to 0.9%, greater than 0 to 0.6%, 0.01 to 0.55%, 0.05 to 0.53%, 0.1 to 0.5%, or 0.15 to 0.4%, in total.

Meanwhile, the recycled bis(2-hydroxyethyl) terephthalate (r-BHET) contained in the recycled raw material composition according to the present invention is formed by the depolymerization of waste polyester. It may be a compound formed as an intermediate in the procedure of preparing a polyester by polymerizing ethylene glycol and terephthalic acid or an ester thereof. The recycled bis(2-hydroxyethyl) terephthalate (r-BHET) can have high purity as the content of impurities is controlled to a specific level or less as described above. Thus, it can have physical properties equivalent to those of virgin bis(2-hydroxyethyl) terephthalate (virgin BHET). In addition, the recycled bis(2-hydroxyethyl) terephthalate (r-BHET) has excellent crystallinity, thereby having a high melting point, and may have excellent quality such as color.

Specifically, the purity of the recycled bis(2-hydroxyethyl) terephthalate (r-BHET) may be calculated by measuring the fraction (%) of the peak area of BHET out of the total peak area in a spectrum of the recycled raw material composition obtained using high-performance liquid chromatography (HPLC).

For example, the recycled raw material composition according to the present invention may have a peak area fraction of bis(2-hydroxyethyl) terephthalate of 92% or more when analyzed by high-performance liquid chromatography (HPLC). Specifically, the peak area fraction of bis(2-hydroxyethyl) terephthalate may be 93% or more, 93.5% or more, 94% or more, 94.3% or more, 94.5% or more, 94.8% or more, 95% or more, 95.3% or more, 95.5% or more, 95.8% or more, 96% or more, 98% or more, 99% or more, or 100%. Accordingly, the recycled bis(2-hydroxyethyl) terephthalate contained in the recycled raw material composition can have high purity.

In addition, the recycled raw material composition may have a peak area fraction of monohydroxyethyl terephthalate (MHET) of 2% or less, 1.8% or less, 1.5% or less, 1.4% or less, 1.2% or less, 1% or less, 0.99% or less, or 0.95% or less, when analyzed by high-performance liquid chromatography (HPLC). Specifically, the peak area fraction of monohydroxyethyl terephthalate (MHET) may be 0 to 2%, greater than 0 to 1.9%, 0.5 to 1.5%, or 0.8 to 1.1%.

Meanwhile, as the content of bis(2-hydroxyethyl) isophthalate and/or diethylene glycol ester compounds in the recycled raw material composition increases, the thermal resistance properties such as melting point (Tm) of a final polymer linearly decrease. An acetate-based ester compound acts as a terminator that inhibits the growth of polymer chains. As its content increases, the thermal resistance properties of a final polymer decrease exponentially. A correlation can be derived using this relationship to predict the thermal resistance properties of a polymer prepared using a recycled raw material composition (specifically, recycled bis(2-hydroxyethyl) terephthalate).

For example, the recycled raw material composition according to the present invention may have a thermal property drop index (TDI) of 6.0 or less as defined by the following Equation 1, when analyzed by high-performance liquid chromatography (HPLC).

[Equation 1]

$$\mathrm{TDI} = [\text{DEG-ester-1}] + ([\text{DEG-ester-2}] \times 2) + \exp\text{^}[\text{HA-ester}] + \exp\text{^}[\text{BHEI}]$$

In Equation 1, DEG-ester-1 is the peak area fraction (%) of 2-hydroxyethyl[2-(2-hydroxyethoxy)ethyl] terephthalate, DEG-ester-2 is the peak area fraction (%) of bis[2-(2-hydroxyethoxy)ethyl]benzene-1,4-dicarboxylate, HA-ester is the peak area fraction (%) of 2-hydroxyethyl(2-acetoxyethyl) terephthalate, and BHEI is the peak area fraction (%) of bis(2-hydroxyethyl) isophthalate.

In Equation 1, it is calculated by taking only the numerical values excluding the units of these parameters.

Specifically, if the thermal property drop index (TDI) defined by Equation 1 is 5.8 or less, it is possible to more effectively prevent the thermal resistance properties of a polymer from deteriorating by DEG-ester-1, DEG-ester-2, HA-ester, and BHEI in the preparation of the polymer (e.g., a recycled polyester resin). For example, the thermal property drop index (TDI) may be 5.5 or less, 5.4 or less, 5.2 or less, 5.0 or less, 4.9 or less, 4.8 or less, 4.5 or less, 4.3 or less, or 4.0 or less (specifically, 0 to 5.5, 0.5 to 5.0, or 1.0 to 4.0).

The recycled raw material composition according to the present invention may have a yellow index (YID) of 5.0 or less when analyzed for a solution (sample recycled raw material solution) dissolved in dimethylformamide at a concentration of 25% by weight. Specifically, the yellow index (YID) may be 4.9 or less, 4.5 or less, 4.3 or less, 4.0 or less, 3.8 or less, 3.5 or less, 3.3 or less, 3.0 or less, 2.5 or less, or 2.3 or less. As it has such a yellow index (YID), the recycled raw material composition according to the present invention can contribute to preparing a polymer (recycled polyester resin) having excellent color characteristics (e.g., transparency).

**Process for preparing a recycled raw material composition**

The process for preparing a recycled raw material composition according to the present invention is a process for preparing a recycled raw material composition with a minimized content of impurities as described above, in which the polarity of a solvent used in the depolymerization procedure is adjusted, and then a cooling crystallization procedure is carried out. Specifically, the process for preparing a recycled raw material composition according to the present invention comprises (1) depolymerizing waste polyester by a glycolysis reaction to obtain a reactant comprising crude bis(2-hydroxyethyl) terephthalate (crude-BHET); (2) treating the reactant with an ion-exchange resin; (3) adding water to the reactant obtained by treatment with the ion-exchange resin to adjust the polarity of the solvent contained in the reactant; (4) cooling the reactant to which water has been added to crystallize it; and (5) performing pressurized filtration of the crystallized material obtained through the crystallization to obtain a product comprising recycled bis(2-hydroxyethyl) terephthalate. Hereinafter, this will be described in detail.

In the present invention, a reactant may refer to a product obtained through each step.

<u>Step (1): Depolymerization of waste polyester</u>

According to the present invention, step (1) is a step of depolymerizing waste polyester by a glycolysis reaction to obtain a reactant (a) comprising crude bis(2-hydroxyethyl) terephthalate (crude-BHET).

The waste polyester subjected to depolymerization may be obtained by pretreating waste products discarded after they have been used by consumers. The waste products may be beverage bottles, fabrics, films, cases, boxes, partitions, shelves, protective panels, packaging materials, building materials, and interior and exterior materials that comprise a polyester.

The polyester contained in the various wastes products may be obtained through a (co)polymerization reaction of one or more commonly known acid components and one or more commonly known alcohol components. The acid component may specifically comprise at least one selected from the group consisting of terephthalic acid, isophthalic acid, dimethyl terephthalic acid, dimethyl terephthalate, naphthalenedicarboxylic acid, orthophthalic acid, adipic acid, azelaic acid, sebacic acid, and decanedicarboxylic acid. The alcohol component may specifically comprise at least one selected from the group consisting of ethylene glycol, 1,3-propanediol, 1,2-octanediol, 1,3-octanediol, 2,3-butanediol, 1,3-butanediol, 1,4-butanediol, 1,5-pentanediol, neopentyl glycol, 2-butyl-2-ethyl-1,3-propanediol, 2,2-diethyl-1,5-pentanediol, 2,4-diethyl-1,5-pentanediol, 3-methyl-1,5-pentanediol, 1,1-dimethyl-1,5-pentanediol, 1,2-cyclohexanedimethanol, 1,3-cyclohexanedimethanol, 1,4-cyclohexanedimethanol, diethylene glycol, isosorbide, and 2,2,4,4-tetramethyl-1,3-cyclobutanediol.

The pretreatment may be carried out by removing other plastics, metals, and foreign substances mixed in the various wastes, washing them, and then crushing them through a crusher. As a result of the pretreatment, the waste polyester may have a flake form. In addition, the waste polyester may have a fine structure like a fiber.

Step (1) of depolymerizing waste polyester may comprise (1-1) depolymerizing (first depolymerizing) the waste polyester by a first glycolysis reaction to obtain a first reactant (a-1); and (1-2) depolymerizing (second depolymerizing) the first reactant (a-1) by a second glycolysis reaction to obtain a second reactant (a-2).

Specifically, step (1-1) may comprise a procedure of carrying out a chemical reaction for primarily severing the polymer chains of the waste polyester with a first glycol-based compound to obtain a first reactant (a-1).

The first glycol-based compound used in the first depolymerization of step (1-1) is not particularly limited, but it may specifically comprise at least one selected from the group consisting of ethylene glycol (monoethylene glycol), propylene glycol, and diethylene glycol.

The feeding amount of the first glycol-based compound in step (1-1) is not particularly limited. Specifically, it may be 1 time or more, 2 times or more, or 3 times or more, and 7 times or less, 5 times or less, or 4 times or less (e.g., 1 to 7 times, 2 to 5 times, or 3 to 4 times) the weight of the waste polyester.

The first depolymerization temperature in step (1-1) is not particularly limited, but it may be 180 to 200°C, specifically, 180 to 195°C, 180 to 193°C, 180 to 190°C, 180 to 188°C, or 180 to 185°C. In addition, the first depolymerization

time is not particularly limited, but it may be 1 to 4 hours, 1 to 3 hours, or 1 to 2 hours from the time when the temperature required for the first depolymerization is reached. As the first depolymerization temperature and time are each within the above ranges, the first glycolysis reaction of the waste polyester can be carried out smoothly, while minimizing the formation of side reactants such as diethylene glycol ester compounds.

The first depolymerization in step (1-1) may be carried out in the presence of a catalyst that activates the first glycolysis reaction. The catalyst is not particularly limited as long as it is a commonly known catalyst, but it may specifically comprise a metal acetate, an anhydride thereof, or a hydride thereof. More specifically, the catalyst may be at least one selected from the group consisting of zinc acetate, sodium acetate, cobalt acetate, and manganese acetate, a hydrate thereof, or anhydride thereof.

The feeding amount (the amount used) of the catalyst in step (1-1) is not particularly limited, but it may specifically be 0.01 to 5 parts by weight, 0.05 to 3 parts by weight, 0.1 to 2 parts by weight, 0.15 to 1 part by weight, 0.2 to 0.6 part by weight, or 0.2 to 0.4 part by weight, relative to 100 parts by weight of the waste polyester.

For example, the first glycolysis reaction carried out in step (1-1) may be a reaction of waste polyester and ethylene glycol in the presence of zinc acetate hydrate.

Step (1-2) may comprise a procedure of carrying out a chemical reaction for secondarily severing the first reactant (a-1) obtained in step (1-1) with a second glycol-based compound to obtain a second reactant (a-2). The second reactant (a-2) may refer to a reactant (a) obtained through step (1).

The second glycol-based compound used in the second depolymerization of step (1-2) is not particularly limited, but it may specifically comprise at least one selected from the group consisting of ethylene glycol (monoethylene glycol), propylene glycol, and diethylene glycol. The second glycol-based compound may be derived from the first depolymerization procedure of step (1-1) or may be further added during the second depolymerization procedure of step (1-2).

The feeding amount (the additional amount used in the second depolymerization) of the second glycol-based compound in step (1-2) is not particularly limited. Specifically, it may be 1 time or more, 2 times or more, or 3 times or more, and 7 times or less, 5 times or less, or 4 times or less (e.g., 1 to 7 times, 2 to 5 times, or 3 to 4 times) the weight of the waste polyester.

The second depolymerization temperature in step (1-2) is not particularly limited, but it may be 150 to 170°C. Specifically, it may be 150 to 165°C, 150 to 163°C, 150 to 160°C, 150 to 158°C, or 150 to 155°C. In addition, the second depolymerization time is not particularly limited, but it may be 1 to 4 hours, 1 to 3 hours, or 1 to 2 hours from the time when the temperature required for the second depolymerization is reached. As the temperature and time of the second depolymerization are each within the above ranges, the second depolymerization of the first reactant (a-1) is carried out smoothly, while minimizing the formation of impurities such as diethylene glycol ester compounds and the like.

The second depolymerization in step (1-2) may be carried out in the presence of a catalyst that activates the second glycolysis reaction. The catalyst may be derived from the first depolymerization procedure of step (1-1) or may be further added during the second depolymerization procedure of step (1-2). The description of the catalyst is the same as the description of the catalyst in step (1-1) above; thus, a detailed description is omitted.

As depolymerization is carried out through steps (1-1) and (1-2), a reactant (a) comprising crude bis(2-hydroxyethyl) terephthalate (crude-BHET) can be obtained in high yield.

Meanwhile, the process for preparing a recycled raw material composition according to the present invention may further comprise, before carrying out step (2) described below, cooling the reactant (a) obtained through step (1) and performing solid-liquid separation thereof to increase the efficiency of removing impurities.

Specifically, in the cooling and solid-liquid separation, the reactant (a) is cooled through a reduced pressure flash and then subjected to solid-liquid separation through a pressurized filtration procedure using a filter aid. As a result, the reactant (a) can be converted to a liquid reactant (b). As the step of cooling and solid-liquid separation is further carried out, solid foreign matters such as particulates and insoluble organic substances contained in the first reactant (a) are removed, thereby increasing the yield and purity of the final product (recycled raw material composition).

The temperature at which the first reactant (a) is cooled through the reduced pressure flash is not particularly limited, but it may specifically be 150°C or lower, 140°C or lower, 135°C or lower, 130°C or lower, 125°C or lower, 120°C or lower, or 115°C or lower, and 50 °C or higher, 70 °C or higher, 80 °C or higher, 100°C or higher, 105°C or higher, or 110°C or higher (e.g., 100 to 135°C, 105 to 125°C, or 110 to 120°C).

The pressure for carrying out the reduced pressure flash is not particularly limited, but it may specifically be 200 Torr or less, 150 Torr or less, 100 Torr or less, 50 Torr or less, or 30 Torr or less, and 5 Torr or more, 8 Torr or more, 10 Torr or more, or 15 Torr or more (e.g., 5 to 200 Torr, 10 to 100 Torr, or 15 to 50 Torr).

The filter aid used for the solid-liquid separation is not particularly limited as long as it is commonly known, but it may specifically comprise at least one selected from the group consisting of diatomaceous earth, perlite, and asbestos powder.

Step (2): Treatment with an ion-exchange resin

According to the present invention, step (2) is a step of treating the reactant (a) (if the cooling and solid-liquid separation are carried out, the reactant (a) becomes a liquid reactant (b)) with an ion-exchange resin. Specifically, the treatment may be carried out by passing the reactant (a) through an ion-exchange resin or adding an ion-exchange resin to the reactant (a). As the above procedure is carried out, ionic impurities contained in the reactant (a) may be removed to obtain a reactant (c) with high purity.

The ion-exchange resin may be a cation-exchange resin, an anion-exchange resin, an amphoteric ion-exchange resin, a chelate resin, or a combination thereof, commonly known.

The cation-exchange resin may specifically comprise a strongly acidic cation-exchange resin having a sulfonic acid group (-$SO_3H$) or a weakly acidic cation-exchange resin having a carboxyl group (-COOH). The anion-exchange resin may comprise a strongly basic anion-exchange resin in the form of a quaternary ammonium salt or a weakly basic anion-exchange resin having a primary to tertiary amino group. The chelate resin may be a polymer resin having a reactive functional group such as acetate or phosphate that chelates metal ions such as sodium, copper, nickel, zinc, and manganese.

When the treatment is carried out by adding the ion-exchange resin to the reactant (a), the feeding amount (the amount used) of the ion-exchange resin is not particularly limited, but it may specifically be 1 time or more, 2 times or more, 3 times or more, or 5 times or more, and 20 times or less, 15 times or less, 10 times or less, or 8 times or less (e.g., 1 to 20 times, 2 to 15 times, 3 to 10 times, or 5 to 8 times) the weight of the catalyst used in the depolymerization of step (1). In addition, the feeding amount (the amount used) of the ion-exchange resin may be 1 part by weight or more, 2 parts by weight or more, 3 parts by weight or more, or 5 parts by weight or more, and 50 parts by weight or less, 20 parts by weight or less, 15 parts by weight or less, 10 parts by weight or less, or 7 parts by weight or less (e.g., 1 to 50 parts by weight, 3 to 20 parts by weight, or 5 to 10 parts by weight), relative to 100 parts by weight of the waste polyester of step (1).

When the treatment is carried out by passing the reactant (a) through the ion-exchange resin, the ion-exchange resin may be in the form of particles having a predetermined size. Specifically, the treatment for removing ionic impurities may be carried out by passing the liquid reactant (b) through a column filled with ion-exchange resin particles having a particle size of 0.3 to 1.5 mm, 0.5 to 1.3 mm, or 0.7 to 1.0 mm.

In the reactant (c) obtained by the treatment with an ion-exchange resin, the polarity of the solvent can be controlled through step (3) described below.

Step (3): Control of solvent polarity

According to the present invention, step (3) is a step of adding water to the reactant (c) obtained by treatment with the ion-exchange resin in step (2) to adjust the polarity of the solvent contained in the reactant (c). When such step (3) is carried out, crystal growth of the recycled bis(2-hydroxyethyl) terephthalate is smoothly achieved in the cooling crystallization procedure of step (4) described below, which significantly increases the pressurized filtration efficiency in step (5) described below and makes possible the separation of BHET analogues (e.g., BHEI) that are hardly separated due to their similar structure to that of bis(2-hydroxyethyl) terephthalate (BHET). As a result, a recycled raw material composition (i.e., recycled bis(2-hydroxyethyl) terephthalate) with high purity can be prepared.

Specifically, if the crystal growth of bis(2-hydroxyethyl) terephthalate (BHET) is successfully achieved in the cooling crystallization procedure of step (4), the separation efficiency of compounds with a low melting point and impurities such as chromophore molecules can be increased. Therefore, it is necessary to ensure that the crystals of bis(2-hydroxyethyl) terephthalate (BHET) grow well. Here, the crystal growth (crystal formation) of bis(2-hydroxyethyl) terephthalate (BHET) remarkably varies depending on the degree of polarity of the solvent contained in the reactant (c) subjected to cooling crystallization. Thus, it is very important to control the polarity of the solvent contained in the reactant (c) obtained through the treatment with an ion-exchange resin before the cooling crystallization procedure.

Accordingly, in the present invention, water is added to the reactant (c) obtained through step (2) to optimally control the polarity of the solvent contained in the reactant (c), thereby allowing the crystals of bis(2-hydroxyethyl) terephthalate (BHET) to grow well in the cooling crystallization procedure of step (4) described below.

According to the present invention, the amount of water added to the reactant (c) obtained by the treatment with an ion-exchange resin is not particularly limited, but it may be 25 to 80% by weight based on the total weight of the solvents, excluding the crude-BHET from the reactant (d) to which water has been added (specifically, the solvents contained in the component, excluding the crude-BHET from the reactant (d)). That is, water may be added to the reactant (c) obtained by the treatment with an ion-exchange resin such that the weight of water is 25 to 80% by weight of the total weight of the solvents contained in the reactant (d) to which water has been added. Specifically, the amount of water added may be 26 to 79% by weight, 27 to 78% by weight, 28 to 77% by weight, 29 to 76% by weight, 30 to 75% by weight, 35 to 75% by weight, 40 to 75% by weight, or 45 to 75% by weight, based on the total weight of the solvents (solvents with controlled polarity). As the amount of water added is within the above range, the pressurized filtration efficiency can be increased while the crystal

growth of bis(2-hydroxyethyl) terephthalate (BHET) is expedited.

According to the present invention, the solvent contained in the reactant (c) may comprise a glycol-based solvent. For example, the reactant (c) may comprise the first glycol compound and/or the second glycol compound used for the first glycolysis reaction and/or the second glycolysis reaction as a glycol solvent. As water is added to the reactant (c), the polarity of the solvents contained in the reactant (c) can be controlled. Specifically, the solvents with controlled polarity may comprise water and a glycol-based solvent (e.g., ethylene glycol (monoethylene glycol), propylene glycol, diethylene glycol, or a combination thereof). The mixing ratio of the water and the glycol-based solvent contained in the solvents with controlled polarity is not particularly limited, but it may be a weight ratio of 25:75 to 80:20, specifically, a weight ratio of 28:72 to 80:20, 29:71 to 80:20, 30:70 to 80:20, 30:70 to 78:22, or 30:70 to 75:25. As the mixing ratio is within the above range, the polarity of the solvents contained in the reactant (c) can be optimized to the desired level.

As water is added as described above, a reactant (d) comprising solvents with controlled polarity can be obtained.

Step (4): Cooling crystallization

According to the present invention, step (4) is a step of cooling the reactant (d) to which water has been added in step (3) to crystallize it. Specifically, as the temperature of the reactant (d) in which the polarity of the solvents has been controlled is lowered to perform crystallization, a crystallized material (e) comprising bis(2-hydroxyethyl) terephthalate (BHET) having crystallinity can be obtained.

The temperature for the crystallization of the reactant (d) is not particularly limited, but it may specifically be 70°C or lower, 60°C or lower, 50°C or lower, 40°C or lower, 30°C or lower, or 25°C or lower, and may be 0°C or higher, 5°C or higher, 10°C or higher, 15°C or higher, or 20°C or higher. For example, the temperature for the crystallization of the reactant (d) may be room temperature (20 $\pm$ 5°C).

As the cooling crystallization is carried out, an acetate-based ester compound and diethylene glycol ester compounds as impurities can be efficiently removed.

Step (5): Pressurized filtration

According to the present invention, step (5) is a step of performing pressurized filtration of the crystallized material (e) obtained in step (4) to obtain a product (f) comprising recycled bis(2-hydroxyethyl) terephthalate. Specifically, the product (f) may be a cake comprising recycled bis(2-hydroxyethyl) terephthalate.

The pressure for pressurized filtration of the crystallized material (e) is not particularly limited, but it may specifically be 0.1 to 21 bar, 0.5 to 10 bar, or 1 to 5 bar. In addition, the temperature for the pressurized filtration is not particularly limited, but it may specifically be 5 to 35°C, 10 to 30°C, or 15 to 25°C.

The filter used for the pressurized filtration is not particularly limited, but it may specifically be a Nutsche filter or a filter press.

The pressurized filtration using a Nutsche filter may comprise a procedure in which the crystallized material is put into the Nutsche filter, and an inert gas such as nitrogen is injected and pressurized to primarily separate the solvents and the solid (cake). In addition, it may further comprise a procedure in which water is injected into the Nutsche filter to wash out the residual solvent remaining in the solid (cake), and inert gas is injected and pressurized to secondarily separate the solid (cake) and water. Thereafter, the solid (cake) thus obtained may be subjected to a drying and cooling procedure.

The pressurized filtration using a filter press may comprise a procedure in which a filtration chamber is created with a filter plate having a filter surface, a filter cloth, and a cover, and the crystallized material (e) is pressurized between the filter cloth and the cover at a high pressure to carry out the solid-liquid separation.

As the pressurized filtration is carried out, an acetate-based ester compound, diethylene glycol ester compounds, and BHET oligomers as impurities can be efficiently removed.

Meanwhile, since the crystallized material (e) is obtained through step (3) of controlling the polarity of the solvents and step (4) of cooling crystallization, it can have excellent pressurized filtration performance. For example, the pressurized filtration rate of the crystallized material (e) may be 100 L/minute or more, specifically, 105 L/minute or more, 115 L/minute or more, 120 L/minute or more, 125 L/minute or more, 140 L/minute or more, 150 L/minute or more, 160 L/minute or more, 170 L/minute or more, 180 L/minute or more, 200 L/minute or more, 220 L/minute or more, 250 L/minute or more, or 270 L/minute or more (e.g., 100 to 300 L/minute, 120 to 290 L/minute, 170 to 280 L/minute, or 220 to 280 L/minute).

According to the present invention, the product (f) obtained by the pressurized filtration may have a peak area fraction of oligomers of dimers or higher of 10.0% or less in total (the sum of the peak area fractions of oligomers of dimers or higher is 10.0% or less), when analyzed by high-performance liquid chromatography (HPLC). Specifically, the peak area fraction of oligomers of dimers or higher (the peak area fractions of dimers of BHET and the peak area fractions of trimers of BHET) may be 9% or less, 8.5% or less, 8% or less, 7.5% or less, 7% or less, 6.5% or less, 6% or less, 5.5% or less, 5.1% or less, 4.5% or less, 4% or less, 3.7% or less, 3.4% or less, or 3% or less (e.g., 0 to 10%, greater than 0 to 8%,

0.1 to 6.5%, or 0.2 to 5.2%), in total.

Specifically, the product (f) obtained by the pressurized filtration may have a peak area fraction of dimer oligomers (e.g., dimers of BHET) of 9% or less, 8% or less, 7% or less, 6% or less, 5% or less, 4.5% or less, 4.4% or less, 4% or less, 3.8% or less, 3.5% or less, 3% or less, 2.9% or less, or 2.7% or less (e.g., 0 to 9%, greater than 0 to 7.5%, 0.1 to 6.5%, or 0.2 to 5%), when analyzed by high-performance liquid chromatography (HPLC).

In addition, the product (f) obtained by the pressurized filtration may have a peak area fraction of trimer oligomers (e.g., trimers of BHET) of 1% or less, 0.99% or less, 0.95% or less, 0.9% or less, 0.8% or less, 0.7% or less, 0.65% or less, 0.6% or less, 0.55% or less, 0.5% or less, 0.4% or less, 0.3% or less, or 0.2% or less (e.g., 0 to 9%, greater than 0 to 7.5%, 0.1 to 6.5%, or 0.2 to 5%), when analyzed by high-performance liquid chromatography (HPLC).

Step (6): Distillation

The process for preparing a recycled raw material composition according to the present invention may further comprise distilling the product (f) obtained through step (5) for further purification. Specifically, step (6) of distilling the product (f) may comprise (6-1) subjecting the product to vacuum distillation; and (6-2) subjecting the product obtained by vacuum distillation in step (6-1) to thin film evaporation.

According to the present invention, step (6-1) is a step of subjecting the product (f) obtained through step (5) to vacuum distillation to remove unreacted glycol compounds (e.g., ethylene glycol and diethylene glycol) contained in the product (f).

A glass distillation apparatus or a rotary evaporator may be used for the vacuum distillation in step (6-1).

The vacuum distillation conditions in step (6-1) are not particularly limited, but it may specifically be carried out at a temperature of 150°C or lower under a pressure of 0.1 to 200 Torr. More specifically, the pressure for carrying out the vacuum distillation may be 0.1 to 150 Torr, 0.2 to 100 Torr, 0.3 to 50 Torr, or 0.5 to 30 Torr. In addition, the temperature for carrying out the vacuum distillation may be 90°C or higher, 100°C or higher, or 110°C or higher, and 145°C or lower, 140°C or lower, or 135°C or lower (e.g., 90 to 150°C, 100 to 145°C, 120 to 135°C, or 100 to 130°C).

The unreacted glycol-based compound removed through the vacuum distillation can be recovered and reused in the depolymerization procedure of step (1), thereby increasing the economic efficiency of the depolymerization process.

According to the present invention, step (6-2) is a step of subjecting the product (g) obtained by vacuum distillation to thin film evaporation to remove oligomers of dimers or higher (e.g., BHET dimers and BHET trimers) contained in the product (f).

A thin-film evaporator comprising an evaporator, a wiper rotor, and a condenser may be used for the thin film evaporation in step (6-2).

The conditions for thin film evaporation in step (6-2) are not particularly limited, but it may specifically be carried out at a temperature of 150 to 250°C under a pressure of 0.005 to 5 Torr. More specifically, the pressure for carrying out the thin film evaporation may be 0.005 to 4.5 Torr, 0.01 to 4 Torr, 0.05 to 3 Torr, or 0.07 to 1.5 Torr. In addition, the temperature for carrying out the thin film evaporation (internal thin film temperature of the thin film evaporator) may be 180 to 240°C, 185 to 230°C, 190 to 225°C, 195 to 220°C, or 200 to 220°C.

The final product (h) obtained through this distillation procedure, i.e., the recycled raw material composition, may comprise recycled bis(2-hydroxyethyl) terephthalate with high purity.

**Mode for the Invention**

Hereinafter, the present invention will be described in more detail with reference to embodiments. However, these examples are provided only for illustration purposes, and the present invention is not limited thereto.

**[Example 1]**

A first reactor made of stainless steel (SUS) was charged with 1,000 g of a waste polyester resin, 2,000 g of ethylene glycol, and 10 g of zinc acetate anhydride. The temperature inside the first reactor was raised to 180°C, and a first depolymerization (first glycolysis reaction) was carried out for 2 hours to obtain a first reactant (a-1). Subsequently, the first reactant (a-1) thus obtained was transferred to a second reactor and cooled to 150°C. Then, 2,000 g of ethylene glycol was further charged to the second reactor, and a second depolymerization (second glycolysis reaction) was carried out for 2 hours, while the temperature of the second reactor was maintained at 150°C, to obtain a second reactant (a-2) comprising crude bis(2-hydroxyethyl) terephthalate (crude-BHET).

The second reactant (a-2) thus obtained was cooled to 120°C through reduced pressure flash, and 16 g of a filter aid (Celite™ 545) was added thereto, followed by pressurized filtration to carry out the solid-liquid separation, to obtain a liquid reactant (b).

Next, the liquid reactant (b) was passed through a column filled with an ion-exchange resin (BC107(H) of Bonlite)

to remove ionic impurities contained in the liquid reactant (b) to obtain a mixture (reactant (c)) comprising crude bis(2-hydroxyethyl) terephthalate (crude-BHET) and ethylene glycol.

Next, water (DIW) was added to the mixture (reactant (c)) to adjust the polarity of the solvent contained in the mixture (reactant (c)). The temperature of the mixture (reactant (d)) to which water had been added was cooled to room temperature in a 10-liter cooling crystallizer to crystallize it. In such an event, water was added (fed) to the mixture (reactant (c)) such that the content of water (DIW) in the solvent components, other than crude-BHET, of the mixture (reactant (d)) was 40% by weight.

The crystallized material (e) obtained through the crystallization was subjected to solid-liquid separation by pressurized Nutsche filtration to obtain a cake comprising recycled bis(2-hydroxyethyl) terephthalate (r-BHET) as a product (f).

The product (f) thus obtained was transferred to a 10-liter distillation apparatus, and vacuum distillation was carried out at 130°C to remove (recover) unreacted ethylene glycol. Subsequently, the product (g) from which ethylene glycol had been removed was subjected to thin film evaporation at 220°C and 0.08 Torr in a thin film evaporator (VKL70-4S of VTA) to remove oligomers of dimers or higher to obtain a recycled raw material composition as a final product (h).

**[Example 2]**

A final product (h) was obtained through the same procedure as in Example 1, except that water was added to the mixture (reactant (c)) such that the content of water in the solvent components, other than crude-BHET, of the mixture (reactant (d)) was 30% by weight.

**[Example 3]**

A final product (h) was obtained through the same procedure as in Example 1, except that water was added to the mixture (reactant (c)) such that the content of water in the solvent components, other than crude-BHET, of the mixture (reactant (d)) was 50% by weight.

**[Example 4]**

A final product (h) was obtained through the same procedure as in Example 1, except that water was added to the mixture (reactant (c)) such that the content of water in the solvent components, other than crude-BHET, of the mixture (reactant (d)) was 75% by weight.

**[Example 5]**

A final product (h) was obtained through the same procedure as in Example 1, except that water was added to the mixture (reactant (c)) such that the content of water in the solvent components, other than crude-BHET, of the mixture (reactant (d)) was 88% by weight.

**[Comparative Example 1]**

A final product (h) was obtained through the same procedure as in Example 1, except that water was not added to the mixture (reactant (c)).

**[Comparative Example 2]**

A final product (h) was obtained through the same procedure as in Example 1, except that water was added to the mixture (reactant (c)) such that the content of water in the solvent components, other than crude-BHET, of the mixture (reactant (d)) was 20% by weight.

**[Comparative Example 3]**

A final product (h) was obtained through the same procedure as in Example 1, except that ethanol, instead of water, was added to the mixture (reactant (c)).

**[Test Example]**

The materials obtained in Examples 1 to 5 and Comparative Examples 1 to 3 were each tested by the following

methods. The results are shown in Table 1 below.

**(1) High-performance liquid chromatography (HPLC)**

0.01 g of a sample (final product (h)) was diluted in 20 ml of methanol, which was analyzed by high-performance liquid chromatography (HPLC) (model: Waters e2695, column: C18 (4.6 × 250 mm), 5 μm, UV detector: 242 nm, injection volume: 10 μl, eluent (gradient) A: $H_2O$ + $H_3PO_4$, B: acetonitrile). Thereafter, the peak area fractions (%) of the following components among the total peak area of HPLC were obtained.

- MHET: monohydroxyethyl terephthalate
- BHET: bis(2-hydroxyethyl) terephthalate,
- BHEI: bis(2-hydroxyethyl) isophthalate
- DEG-ester-1: 2-hydroxyethyl[2-(2-hydroxyethoxy)ethyl] terephthalate
- DEG-ester-2: bis[2-(2-hydroxyethoxy)ethyl]benzene-1,4-dicarboxylate
- HA-ester: 2-hydroxyethyl(2-acetoxyethyl) terephthalate
- Dimer: BHET dimer
- Trimer: BHET trimer

**(2) TDI**

HPLC analysis of a sample (final product (h)) was carried out using the test method of Section (1) above, and the thermal property drop index (TDI) represented by the following Equation 1 was calculated.

[Equation 1]

$$TDI = [DEG\text{-}ester\text{-}1] + ([DEG\text{-}ester\text{-}2] \times 2) + exp\hat{}[HA\text{-}ester] + exp\hat{}[BHEI]$$

In Equation 1, DEG-ester-1 is the peak area fraction (%) of 2-hydroxyethyl[2-(2-hydroxyethoxy)ethyl] terephthalate, DEG-ester-2 is the peak area fraction (%) of bis[2-(2-hydroxyethoxy)ethyl]benzene-1,4-dicarboxylate, HA-ester is the peak area fraction (%) of 2-hydroxyethyl(2-acetoxyethyl) terephthalate, and BHEI is the peak area fraction (%) of bis(2-hydroxyethyl) isophthalate. It is calculated by taking only the numerical values excluding the units of these parameters. Here, exp^ stands for exponential function (e^).

**(3) Yellow index (YID)**

A sample (final product (h)) was dissolved in dimethylformamide at room temperature at a concentration of 25% by weight to prepare a solution. The solution thus prepared was left for 30 minutes, after which its yellow index was measured. Specifically, transmission data were obtained with Illuminuteant D65 using Color Flex EZ of HunterLab at an observer's angle of 2°. The yellow index (YID) value was calculated using a color analyzer in the software.

**(4) Pressurized filtration rate**

The crystallized material (e) was filtered using a filter press (filtration area 0.4 $m^2$, 4 ea filter plates) under a pressure condition of 18 bar to measure the pressurized filtration rate.

◎: filtration rate of 250 L/minute or more

◦: filtration rate of 100 L/minute to less than 250 L/minute

×: filtration rate of less than 100 L/minute

[Table 1]

| | Info. | Ex. 1 | Ex. 2 | Ex. 3 | Ex. 4 | Ex. 5 | C. Ex. 1 | C. Ex. 2 | C. Ex. 3 |
|---|---|---|---|---|---|---|---|---|---|
| DIW/total solvent | Weight ratio | 0.40 | 0.30 | 0.50 | 0.75 | 0.88 | 0.00 | 0.20 | 0.40 (ethanol/ total solvent) |
| HPLC (%) of the product (f) | BHET | 92.10 | 93.40 | 92.70 | 91.82 | 85.20 | 87.20 | 88.40 | 89.46 |
| | BHEI | 0.88 | 0.92 | 0.74 | 0.18 | 0.17 | 1.83 | 1.41 | 1.42 |
| | MHET | 1.43 | 1.10 | 0.98 | 0.92 | 0.38 | 2.38 | 2.22 | 2.75 |
| | DEG-ester-1 | 1.57 | 1.64 | 1.34 | 1.43 | 1.20 | 5.20 | 4.78 | 3.90 |
| | DEG-ester-2 | 0.14 | 0.13 | 0.09 | 0.07 | 0.22 | 0.80 | 0.70 | 0.11 |
| | HA-ester | 0.05 | 0.03 | 0.06 | 0.09 | 0.12 | 1.38 | 1.10 | 1.19 |
| | Dimer | 2.88 | 2.65 | 3.34 | 4.39 | 9.35 | 0.92 | 1.20 | 0.99 |
| | Trimer | 0.23 | 0.12 | 0.34 | 0.65 | 1.90 | 0.18 | 0.13 | 0.11 |
| | Others | 0.72 | 0.01 | 0.40 | 0.44 | 1.46 | 0.11 | 0.04 | 0.07 |
| Pressurized filtration rate of the crystallized material (e) | L/min. | 174.4 | 278.8 | 125.1 | 105.9 | 34.4 | 521.9 | 339.1 | 429.9 |
| | Filtering performance | ○ | ◎ | ○ | ○ | × | ◎ | ◎ | ◎ |
| HPLC (%) of the final product (h) | BHET | 94.86 | 96.10 | 96.67 | 96.40 | 96.30 | 88.20 | 89.40 | 89.28 |
| | BHEI | 0.91 | 0.29 | 0.45 | 0.34 | 0.19 | 1.91 | 1.55 | 1.88 |
| | MHET | 1.47 | 1.13 | 0.92 | 0.95 | 0.38 | 2.38 | 2.22 | 2.77 |
| | DEG-ester-1 | 1.62 | 1.69 | 1.38 | 1.47 | 1.32 | 5.60 | 4.88 | 3.92 |
| | DEG-ester-2 | 0.15 | 0.14 | 0.10 | 0.07 | 0.23 | 0.84 | 0.69 | 0.22 |
| | HA-ester | 0.05 | 0.03 | 0.06 | 0.09 | 0.10 | 1.42 | 1.13 | 1.21 |
| | Dimer | 0.25 | 0.33 | 0.33 | 0.45 | 0.45 | 0.22 | 0.25 | 0.22 |
| | Trimer | 0.02 | 0.04 | 0.05 | 0.05 | 0.59 | 0.04 | 0.05 | 0.02 |
| | Others | 0.67 | 0.25 | 0.04 | 0.17 | 0.61 | 0.63 | 0.65 | 0.48 |
| TDI | 5.4 | 4.3 | 4.2 | 4.1 | 18.2 | 14.1 | 4.1 | 4.31 | |
| YID | 3.6 | 4.9 | 3.1 | 2.2 | 1.7 | 1.7 | 7.6 | 10.1 | |

Referring to Table 1 above, the degree of crystal growth of BHET varied depending on the composition and polarity of the solvents in the cooling crystallization step (4), whereby the removal (separation) performance of low-melting impurities and chromophore molecules varied. Specifically, in the Examples of the present invention, in which the polarity of the solvents was controlled to expedite the crystal growth of BHET, while the solubility of oligomers of dimers or higher was increased, BHET with high purity was obtained while the content of impurities such as HA-ester, DEG-ester-1, DEG-ester-2, dimers, trimers, and the like was minimized.

In such an event, if water is added in excess, the solubility of oligomers of dimers or higher decreases, and the undissolved oligomers act as an obstacle to the crystal growth of BHET, which may increase the amount of microcrystals formed, thereby lowering the pressurized filtration performance (the precipitation effect becomes dominant). Thus, it is desirable to control the amount of water added such that it is not added in excess (see Example 5).

In addition, if water was not added (see Comparative Example 1), or if water was added at a level that failed to control the polarity of the solvent (see Comparative Example 2), the purity of BHET was significantly reduced since the polarity of the solvent was not sufficient to expedite the crystal growth of BHET. In addition, even if the polarity of the solvent was adjusted by using ethanol instead of water (see Comparative Example 3), the purity of BHET was significantly reduced since the change in the polarity of the solvent was not large.

## Claims

1. A recycled raw material composition, which comprises bis(2-hydroxyethyl) terephthalate formed by the depolymerization of waste polyester and has a peak area fraction of an acetate-based ester compound of 1.0% or less when analyzed by high-performance liquid chromatography (HPLC).

2. The recycled raw material composition of claim 1, which has a peak area fraction of a compound comprising bis(2-hydroxyethyl) isophthalate of 1.0% or less when analyzed by high-performance liquid chromatography (HPLC).

3. The recycled raw material composition of claim 1, which has a peak area fraction of diethylene glycol ester compounds of 2.0% or less in total when analyzed by high-performance liquid chromatography (HPLC).

4. The recycled raw material composition of claim 1, which has a peak area fraction of oligomers of dimers or higher of 2.0% or less in total when analyzed by high-performance liquid chromatography (HPLC).

5. The recycled raw material composition of claim 1, wherein the recycled raw material composition has a yellow index (YID) of 5.0 or less when analyzed for a solution as dissolved in dimethylformamide at a concentration of 25% by weight.

6. The recycled raw material composition of claim 1, which has a peak area fraction of bis(2-hydroxyethyl) terephthalate of 92% or more when analyzed by high-performance liquid chromatography (HPLC).

7. The recycled raw material composition of claim 1, which has a thermal property drop index (TDI) of 6.0 or less, as defined by the following Equation 1, when analyzed by high-performance liquid chromatography (HPLC):

[Equation 1]

$$TDI = [DEG\text{-}ester\text{-}1] + ([DEG\text{-}ester\text{-}2] \times 2) + \exp^\wedge[HA\text{-}ester] + \exp^\wedge[BHEI]$$

in Equation 1, DEG-ester-1 is the peak area fraction (%) of 2-hydroxyethyl[2-(2-hydroxyethoxy)ethyl] terephthalate, DEG-ester-2 is the peak area fraction (%) of bis[2-(2-hydroxyethoxy)ethyl]benzene-1,4-dicarboxylate, HA-ester is the peak area fraction (%) of 2-hydroxyethyl(2-acetoxyethyl) terephthalate, and BHEI is the peak area fraction (%) of bis(2-hydroxyethyl) isophthalate, wherein it is calculated by taking only the numerical values excluding the units of these parameters.

8. A process for preparing a recycled raw material composition, which comprises:

(1) depolymerizing waste polyester by a glycolysis reaction to obtain a reactant comprising crude bis(2-hydroxyethyl) terephthalate (crude-BHET);
(2) treating the reactant with an ion-exchange resin;
(3) adding water to the reactant obtained by treatment with the ion-exchange resin to adjust the polarity of the solvent contained in the reactant;
(4) cooling the reactant to which water has been added to crystallize it; and
(5) performing pressurized filtration of the crystallized material obtained through the crystallization to obtain a product comprising recycled bis(2-hydroxyethyl) terephthalate.

9. The process for preparing a recycled raw material composition of claim 8, wherein step (1) comprises:

(1-1) depolymerizing the waste polyester by a first glycolysis reaction at 180 to 200°C to obtain a first reactant; and
(1-2) depolymerizing the first reactant by a second glycolysis reaction at 150 to 170°C to obtain a second reactant.

10. The process for preparing a recycled raw material composition of claim 8, wherein the amount of water added in step (3) is 25 to 80% by weight based on the total weight of the solvents, excluding the crude-BHET from the reactant to which water has been added.

11. The process for preparing a recycled raw material composition of claim 8, wherein the solvent contained in the reactant obtained by treatment with the ion-exchange resin comprises a glycol-based solvent, and

the solvents with controlled polarity comprise water and the glycol-based solvent at a weight ratio of 25:75 to 80:20.

**12.** The process for preparing a recycled raw material composition of claim 8, wherein the pressurized filtration rate of the crystallized material in step (5) is 100 L/minute or more.

**13.** The process for preparing a recycled raw material composition of claim 8, wherein the product obtained in step (5) has a peak area fraction of oligomers of dimers or higher of 10.0% or less in total when analyzed by high-performance liquid chromatography (HPLC).

**14.** The process for preparing a recycled raw material composition of claim 8, which further comprises (6) distilling the product obtained through step (5).

**15.** The process for preparing a recycled raw material composition of claim 14, wherein step (6) comprises:

(6-1) subjecting the product to vacuum distillation; and
(6-2) subjecting the product obtained by vacuum distillation in step (6-1) to thin film evaporation.

**INTERNATIONAL SEARCH REPORT**

International application No. **PCT/KR2024/012341**

**A. CLASSIFICATION OF SUBJECT MATTER**

**C07C 69/84**(2006.01)i; **C07C 67/52**(2006.01)i; **C07C 67/54**(2006.01)i; **C07C 67/03**(2006.01)i; **C08J 11/24**(2006.01)i

According to International Patent Classification (IPC) or to both national classification and IPC

**B. FIELDS SEARCHED**

Minimum documentation searched (classification system followed by classification symbols)

C07C 69/84(2006.01); B01D 9/02(2006.01); B29B 17/02(2006.01); C07C 67/03(2006.01); C07C 67/31(2006.01); C07C 69/82(2006.01); C08J 11/20(2006.01); C08J 11/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폐 폴리에스테르(waste polyester), 비스(2-히드록시에틸)테레프탈레이트(bis(2-hydroxyethyl)terephthalate), 아세테이트계 에스테르 화합물(acetate based ester compound), 디에틸렌글리콜 에스테르 화합물(diethyleneglycol ester compound), 글리콜리시스 반응(glycolysis reaction)

**C. DOCUMENTS CONSIDERED TO BE RELEVANT**

| Category* | Citation of document, with indication, where appropriate, of the relevant passages | Relevant to claim No. |
|---|---|---|
| X | JP 2003-055300 A (IS KK) 26 February 2003 (2003-02-26) See abstract; claims 1-4; and paragraphs [0001]-[0003], [0007], [0024], [0026] and [0029]-[0032]. | 1-4,6-8,10-15 |
| A | | 5,9 |
| X | JP 2006-232701 A (IS KK) 07 September 2006 (2006-09-07) See abstract; claims 1-4; and paragraphs [0058]-[0066]. | 1,3,8,14 |
| A | KR 10-2021-0067554 A (LOTTE CHEMICAL CORPORATION) 08 June 2021 (2021-06-08) See abstract; claims 1-7; and paragraphs [0040]-[0048]. | 1-15 |
| A | KR 10-2021-0067555 A (LOTTE CHEMICAL CORPORATION) 08 June 2021 (2021-06-08) See claims 1-6; and paragraphs [0038]-[0051]. | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 November 2024** | **21 November 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
PCT/KR2024/012341

C. DOCUMENTS CONSIDERED TO BE RELEVANT

| Category* | Citation of document, with indication, where appropriate, of the relevant passages | Relevant to claim No. |
|---|---|---|
| A | JP 2005-330444 A (IS KK) 02 December 2005 (2005-12-02)<br>See claims 1-7; and paragraphs [0069] and [0080]-[0089]. | 1-15 |

Form PCT/ISA/210 (second sheet) (July 2022)

**INTERNATIONAL SEARCH REPORT**
**Information on patent family members**

International application No.
**PCT/KR2024/012341**

| Patent document cited in search report | Publication date (day/month/year) | Patent family member(s) | Publication date (day/month/year) |
|---|---|---|---|
| JP 2003-055300 A | 26 February 2003 | None | |
| JP 2006-232701 A | 07 September 2006 | None | |
| KR 10-2021-0067554 A | 08 June 2021 | None | |
| KR 10-2021-0067555 A | 08 June 2021 | WO 2021-107691 A1 | 03 June 2021 |
| JP 2005-330444 A | 02 December 2005 | None | |

Form PCT/ISA/210 (patent family annex) (July 2022)